# EUROPEAN PATENT SPECIFICATION

(11) **EP 0 430 045 B1**
(45) Date of publication and mention of the grant of the patent: **03.05.1995**
(21) Application number: 90122218.2
(22) Date of filing: 20.11.1990
(51) Int. Cl.: A61K 31/665

(54) **Ascorbic acid tocopheryl phosphate diesters for inhibition of Maillard's reaction**
Ascorbinsäure-Tocopheryl-Phosphatdiester zur Hemmung der Maillard-Reaktion
Diesters phosphates de l'acide ascorbique et du tocophérol pour inhiber la réaction de Maillard

(30) Priority: 21.11.1989 JP 304479/89
(43) Date of publication of application: 05.06.1991
(73) Proprietor: Senju Pharmaceutical Co., Ltd., Osaka-shi, Osaka 541 (JP)
(72) Inventor: Inoue, Jun, Settsu-shi, Osaka-fu (JP)
(74) Representative: Kinzebach, Werner, Dr.

(56) References cited:
- EP-A- 0 127 471
- EP-A- 0 236 120
- EP-A- 0 306 904
- EP-A- 0 324 387
- CHEMICAL ABSTRACTS, vol. 111, n 6, August 1989,p 387 abstract n 45280t ,Columbus, Ohio, US; K. SENOO et al.

## Description

### BACKGROUND OF THE INVENTION

This invention relates to the inhibition of denaturation reaction of proteins by glucose (Maillard's reaction.) More specifically this invention relates to the inhibition of the formation of Amadori rearrangement products which originate from non-enzymatic bond formation between glucose and proteins.

The reaction in which proteins turn brown by reacting non-enzymatically with reductive sugars such as glucose was first reported by Maillard in 1912 [Maillard, L.C., Compt. Rend. Soc. Biol., 72:599 (1912).] Since then, the reaction has been widely recognized by the name of Maillard's reaction in the field of food chemistry. For example, it has been noted that proteins react with glucose in stored or heated food, generate a brown color and finally are denaturated by formation of cross-linkings among molecules.

Later, attentions came to be attracted to reactions of glucose with proteins which may occur in living bodies when Rahbar reported that the level of Hb_{A1c}, a minor component of hemoglobin, was found elevated in red blood cells of diabetic patients [Rahbar, S., Clin. Chim. Acta, 22:296 (1968).] And, through structural analysis of Hb_{A1c}, it has been confirmed that Maillard's reaction occurs in living bodies.

The mechanism of Maillard's reaction in living bodies has been presented by Brownlee et al. [Brownlee, M. et al., Science, 232:1629 (1986).] The reaction proceeds as follows.

At first, the aldehyde group of the open-ring structure of glucose reacts with an amino group in protein molecule to form a Schiff's base. The resulting Schiff's base is unstable and is rapidly converted into Amadori rearrangement product via intra-molecular rearrangement reaction. If this protein is maintained for a long period within the body, the rearranged product undergoes a gradual dehydration reaction to form a new glucose derivative. This derivative then irreversively forms cross-linkings with a variety of molecules including proteins to form bridges among molecules, thus yielding aggregation products of, chiefly, proteins.

This type of product resulting from advanced reactions of glycosylated proteins is usually abbreviated to AGE (Advanced Glycosylation End product.)

In parallel to the formation of AGE, biological adaptibility of the protein is lowered, and the protein becomes less soluble and more resistant to proteases and, in many cases, turns to yellow-brown and fluorescent.

Though observed also in healthy human, Maillard's reaction is markedly noted in those with diabetes mellitus, which is characterized by the elevation of blood glucose. Maillard's reaction is especially notable in proteins with slower rate of metabolic turnover, for example crystallins, which are the structural proteins in the lens, and collagens. While a variety of disorders, for example neuropathy, cataract, nephropathy, retinopathy, arthrosclerosis and atherosclerosis, are noted as complications of diabetes mellitus, these disorders bear a very close resemblance with disorders noted quite frequently in aged human.

It, therefore, is regarded that AGE is also formed gradually from proteins with a slower turnover rate by glycosylation with glucose even under a nomal level of blood sugar.

Upon the said background, efforts have been made in search of compounds which may inhibit Maillard's reaction within living bodies. An example of such efforts has been shown by Brownlee as cited who reported that aminoguanidine inhibits Maillard's reaction in vitro and suppresses AGE formation in arterial walls of diabetic rats in vivo. In Japanese Patent Publication Kokai No. 142114/87, it has been suggested that aminoguanidine, α-hydrazinohistidine and lysine may block the active carbonyl group of Amadori rearrangement products to inhibit AGE formation. It has also been disclosed that different compounds may suppress Maillard's reaction. Such compounds include thiosemicarbazides, 1,3-diaminoguanidine and benzoylhydrazine (Japanese Patent Publication Kokai No. 56614/89), and various derivatives of guanidine (Japanese Patent Publication Kokai No. 83059/89.)

In the patent publications cited above, researches for inhibitors of Maillard's reaction were made using the amount of AGE, the end product of Maillard's reaction, as an index. The present inventor, instead, took the inhibition of formation of Amadori rearrangement product as an index in the investigation. This was based on an estimation that a markedly effective inhibition of Maillard's reaction may be expected by inhibiting the very formation of Amadori rearrangement product, which is the immediate causing factor in protein aggregation process in Maillard's reaction.

Bruggemann et al. [J. Bruggemann et al., Lebensm. Unters. Forsch., 137:137-143 (1968)] and Finot et al. [P.A. Finot et al., Experientia, 24:1097-1099 (1968)] have reported that the amount of ε-N-(furoyl-methyl)-L-lysine hereinafter referred to as "furosine", which is an Amadori rearrangement product resulted from non-enzymatic glycosylation of ε-amino residue of lysine in proteins, may be taken as an index of the non-enzymatic glycosylation of protein molecules. The present inventor made an intensive research for the optimal experimental condition for formation of furosine from protein dissolved in water containing glucose, and, according to the condition thus established, evaluated various compounds for the presence and strength of inhibitory effect on furosine formation.

As a result, the present inventor discovered a potent inhibitory effect in certain compounds which are composed of ascorbic acid and one of tocopherols connected with each other via phosphoric acid ester bonds. The present invention has been accomplished by the investigation based upon this discovery.

### SUMMARY OF THE INVENTION

It, therefore, is a principal object of the present invention to provide pharmaceutical compositions as inhibitors of Maillard's reaction in human body.

It also is specific object to provide pharmaceutical compositions for the treatment or prophylaxis of the diabetic complication disorders neuropathy or arthrosclerosis which develop via Maillard's reaction.

Other objects and advantages of the present invention will become apparent to those skilled in the art as the description proceeds.

Thus, the pharmaceutical composition of the present invention is a pharmaceutical composition comprising in admixture with a pharmaceutically acceptable carrier a compound of the formula (I): 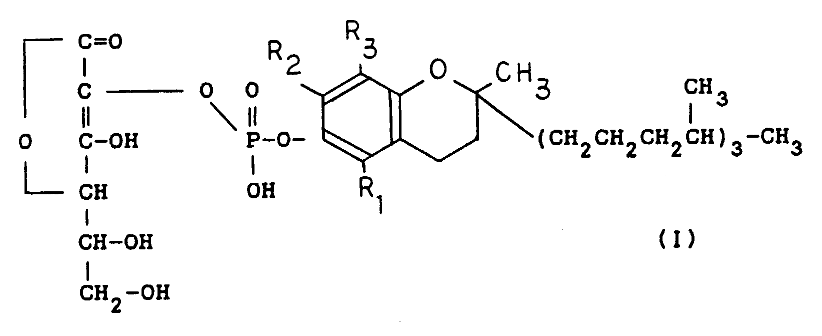
wherein R₁, R₂ and R₃ independently of one another denote hydrogen or methyl, or a pharmaceutically acceptable salt thereof.

### DETAILED DISCUSSION

The examples of pharmaceutically acceptable salts of the compounds represented by the formula (I) include, in particular, alkali metal salts thereof such as sodium salt and potassium salt and alkaline earth metal salts thereof such as calcium salt and magnesium salt.

It, however, is not intended to limit the scope of the present invention by these examples, and salts which are acceptable as pharmaceuticals are included in the scope of the present invention.

It has been disclosed that the compounds represented by the formula (I) have prophylactic and therapeutic effects on cataract and climacteric disturbances, skin beautifying effect, phospholipase A₂ inhibitory effect, antiinflammatory effect, scurf suppressing effect, anti-oxidative effect and anti-ulcer effect in Japanese Patent Publication Kokai Nos. 219295/84, 145019/87, 205091/87, 139114/88, 139972/88 and 270626/88. The disclosures above, however, have failed to offer any suggestion or indication of the presence of an inhibitory effect in those compounds on the non-enzymatic reaction between sugars and proteins. Thus, it is surprising that the compounds represented by the formula (I) have a Maillard's reaction inhibitory effect.

The Maillard's reaction inhibitors of the present invention may be used for the treatment or prophylaxis of a variety of disorders in human body mentioned hereinbefore which may develop via Maillard's reaction. For the purpose, the inhibitors may be administered orally or parenterally. The inhibitors may also be applied topically, for example, in the form of eye drops.

The Maillard's reaction inhibitors represented by the formula (I) (also in the form of pharmaceutically acceptable salts thereof) may be administered orally at a dose of 5 to 2,000 mg/day, more preferably 20 to 1,000 mg/day. For injection, the dose may be 0.5 to 200 mg/day, more preferably 2.0 to 100 mg/day.

For topical application to the eye, the Maillard's reaction inhibitor of the present invention may be applied in the form of eye drops containing the inhibitor at a concentration of 0.05 to 5 w/v %, more preferably 0.2 to 2.5 w/v %.

However, the examples above are not intended to limit the scope of the present invention. A suitable dose may be set according to the type and severity of disorders and schedules of treatment in each case.

The Maillard's reaction inhibitor represented by the formula (I) or a pharmaceutically acceptable salt thereof may be formed into, for example, tablets, pilles, powder, granules or capsules for oral administration, aqueous or non-aqueous solution, suspension or emulsion for injection, or eye drops or eye ointment for ophthalmic topical use.

For preparing pharmaceutical composition of the present invention into the form of tablets, ingredients usually incorporated in tablet preparation may suitably be utilized.

Such ingredients include, for example, diluent bases such as hydroxypropylcellulose, crystalline cellulose, corn starch, polyvinylpyrrolidone, and magnesium metasilicate aluminate, lubricants such as magnesium stearate, disintegrators such as fibrinous calcium gluconate, and solubilizers such as glutamic acid and aspartic acid.

For preparing a pharmaceutical composition of the present invention into the form of aqueous injection, ingredients usually incorporated in injectable preparations may suitably be utilized. Such ingredients include, for example, buffering agents such as phosphates, preservatives such as chlorobutanol, stabilizers such as sodium sulfite, and isotonizers such as sodium chloride.

For preparing a pharmaceutical composition of the present invention into the form of eye drops, ingredients usually incorporated in the formation of eye drops may suitably utilized. Such ingredients include, for example, buffering agents such as phosphates, borates and acetates, preservatives such as chlorobutanol and benzalkonium chloride, stabilizers such as sodium sulfite and sodium edetate, isotonizers such as sodium chloride, potassium chloride and glycerol, and solubilizers such as polysorbate 80 and cyclodextrins.

The inhibitors of Maillard's reaction represented by the formula (I) and pharmaceutically acceptable salts thereof inhibit the very formation of Amadori rearrangement product, the immediate causing factor of cross linkings among protein molecules.

The pharmaceutical compositions of the present invention, accordingly, may be useful for treatment and prophylaxis of diabetic complications, for example coronary heart disease, peripheral circulation disorders, cerebrovascular disorders, neuropathy, nephropathy, arteriosclerosis, arthrosclerosis, cataract and retinopathy, and age-associated disorders such as atherosclerosis, coronary heart disease, cerebrovascular disorders and senile cataract.

The effect of the Maillard's reaction inhibitors of the present invention was determined as follows.

### Pharmacological Test

### Test compounds:

Monopotassium salt of the compound (I) wherein R₁, R₂ and R₃ are all methyl groups (hereinafter referred to as Compound A) was tested.

### Test methods:

Sample solutions as shown below were aseptically prepared from bovine serum albumine (No. A-8022, Sigma)(hereinafter referred to as BSA), 50 mM phosphate buffer solution (PH 7.3) and the test compound and aminoguanidine.

### [Sample solutions]

- Normal sample;: 20 mg/ml BSA in buffer solution
- Control sample:: 20 mg/ml BSA and 50 mM glucose in buffer solution
- Test sample;: 20 mg/ml BSA, 50 mM glucose and 5 mM test compound in buffer solution

The sample solutions were kept for 4 weeks at 37 °C, and the amount of furosine which was formed by non-enzymatic glycosylation was determined by HPLC according to the method of Schleicher et al. [J. Clin. Biochem., 19:81-87 (1981).] Thus, the sample solutions after reaction were dialyzed, and aliquots of 1 ml were lyophylized and then hydrolyzed by the addition of 1 ml of 6 N hydrochloric acid followed by heating at 100 °C for 20 hours. After removal of hydrochloric acid by evaporation, 1 ml of water was added to each sample, and the samples were subjected to filtration using a filter with the pore size of 0.45 »m. The filtrate was used as the sample for HPLC. ODS-120T (Toso) was used for the column and 7 mM phosphoric acid solution was used as the eluant. The absorbance peak whose ratio of peak area at 280 mm/254 mm was 3.9/1 was regarded as the peak corresponding to furosine.

Upon the area of the peak of furosine of each sample, the inhibition rate of furosine formation by the test compound was calculated as follows. Inhibition rate (%)=(c-d)÷(c-n)×100
- c;: peak area of furosine of the control sample
- d;: peak area of furosine of the test sample
- n;: peak area of furosine of the normal sample

### Results:

As shown in Table 1, the test compound exhibited a remarkably potent inhibitory effect in comparison with aminoguanidine, a known inhibitor of Maillard's reaction.

**Table 1**

| Compound: | % inhibition |
|---|---|
| Compound A (monopotassium salt) | 45.1 |
| Aminoguanidine | 7.5 |

The following examples of pharmaceutical compositions are offered for illustrating purposes only. Compounds B and C used therein are as defined below.

| | R₁ | R₂ | R₃ |
|---|---|---|---|
| Compound B: | -CH₃ | -H | -CH₃ |
| Compound C: | -CH₃ | -CH₃ | -H |

### Example 1 Oral tablets

According to the formula below , the ingredients are admixed and formed into tablets by the conventional method. Sugar coatings may optionally be made.

| | |
|---|---|
| Compound A (monopotassium salt) | 100 mg |
| lactose | 80 mg |
| corn starch | 17 mg |
| magnesium stearate | 3 mg |

### Example 2 Oral tablets

According to the formula below, the ingredients are admixed and formed into tablets by the conventional method. Sugar coating may optionally be made.

| | |
|---|---|
| Compound A (sodium salt) | 50 mg |
| corn starch | 90 mg |
| lactose | 30 mg |
| hydroxypropylcellulose | 25 mg |
| magnesium stearate | 5 mg |

### Example 3 Capsules

According to the formula below, the ingredients are admixed, granulated and filled in capsules at an amount of 100 mg/capsule.

| | |
|---|---|
| Compound B (calcium salt) | 25 mg |
| corn starch | 30 mg |
| lactose | 20 mg |
| crystalline cellulose | 24 mg |
| talc | 0.5 mg |
| magnesium stearate | 0.5 mg |

### Example 4 Injection

According to the formula below, the ingredients are admixed by the conventional method to dissolve. The solution is filtered, filled into vials and autoclaved to sterilize.

| | |
|---|---|
| Compound A (disodium salt) | 20 mg |
| chlorobutanol | 5 mg |
| water for injection | to 1 ml |

### Example 5 Eye drops

According to the formula below, the ingredients are admixed by the conventional method to dissolve, and the solution is sterilized by filtration.

| | |
|---|---|
| Compound C (disodium salt) | 0.5 g |
| boric acid | 1.0 g |
| borax | q.s. (to pH 7.0) |
| sodium chloride | 0.25 g |
| disodium edetate | 0.02 g |
| chlorobutanol | 0.2 g |
| polysorbate 80 | 0.2 g |
| sodium sulfite | 0.2 g |
| sterile purified water | to 100 ml |

### Example 6 Eye ointment

According to the formula below, the ingredients are admixed by the conventional method to form eye ointment.

| | |
|---|---|
| Compound A (monopotassium salt) | 0.5 g |
| white vaseline | 100 g |

## Claims

1. The use of at least one compound of formula (I) 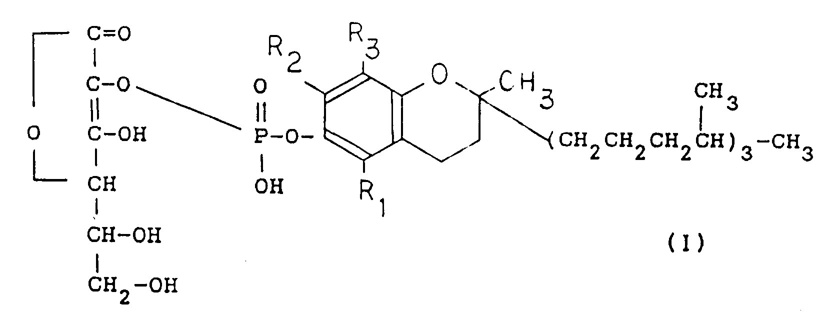 wherein R₁, R₂ and R₃ independently of one another denote hydrogen or methyl, or of a pharmaceutically acceptable salt thereof,
for preparing a pharmaceutical composition for the inhibition of Maillard's reaction in the living body.

2. The use of at least one compound of formula (I) 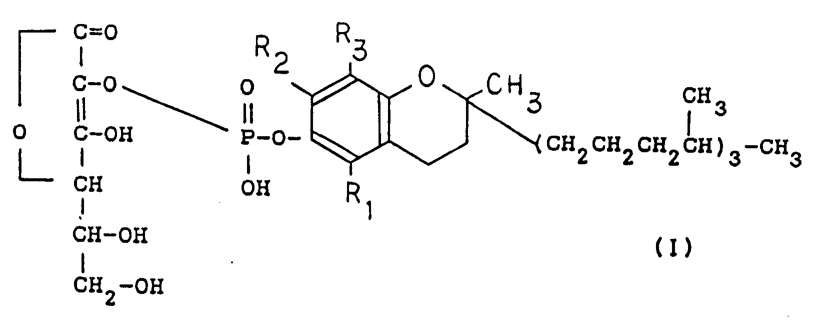 wherein R₁, R₂ and R₃ independently of one another denote hydrogen or methyl, or of a pharmaceutically acceptable salt thereof,
for preparing a pharmaceutical composition for the treatment and prophylaxis of the diabetic complication disorders neuropathy or arthrosclerosis in the human body developing via Maillard's reaction in said human body.

## Patentansprüche

1. Verwendung wenigstens einer Verbindung der Formel (I) 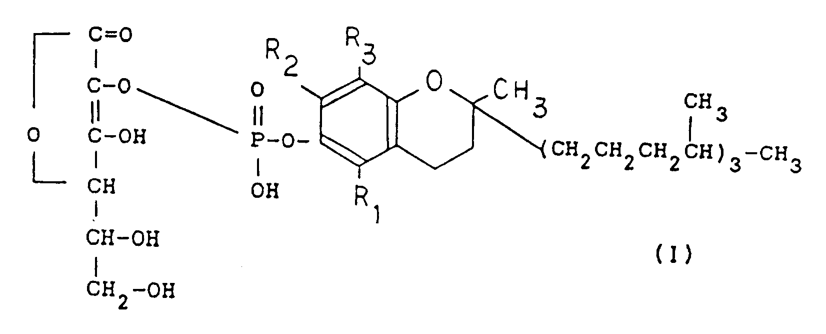 worin R₁, R₂ und R₃ unabhängig voneinander für Wasserstoff oder Methyl stehen, oder eines pharmazeutisch akzeptablen Salzes davon zur Herstellung eines pharmazeutischen Mittels zur Inhibierung der Maillard-Reaktion im lebenden Körper.

2. Verwendung wenigstens einer Verbindung der Formel (I) 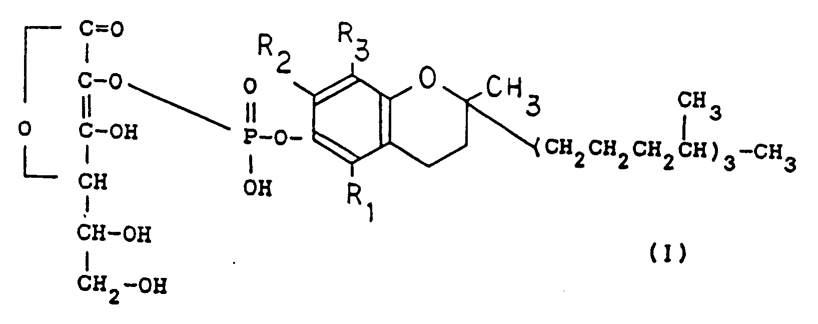 worin R₁, R₂ und R₃ unabhängig voneinander für Wasserstoff oder Methyl stehen oder eines pharmazeutisch akzeptablen Salzes davon zur Herstellung eines pharmazeutischen Mittels zur Behandlung und Prophylaxe der mit diabetischen Komplikationen verbundenen Störungen Neuropathy oder Arthrosklerose im menschlichen Körper, die sich im menschlichen Körper über die Maillard-Reaktion entwickeln.

## Revendications

1. Utilisation d'au moins un composé présentant la formule (I) : 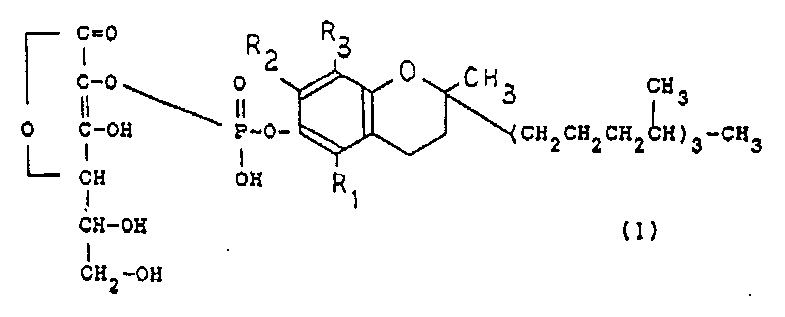 dans laquelle R₁, R₂ et R₃ désignent indépendamment l'un de l'autre un atome d'hydrogène ou un groupe méthyle, ou d'un sel correspondant pharmaceutiquement acceptable, pour le préparation d'une composition pharmaceutique d'inhibition de la réaction de Maillard dans l'organisme vivant.

2. Utilisation d'au moins un composé présentant la formule (I) : 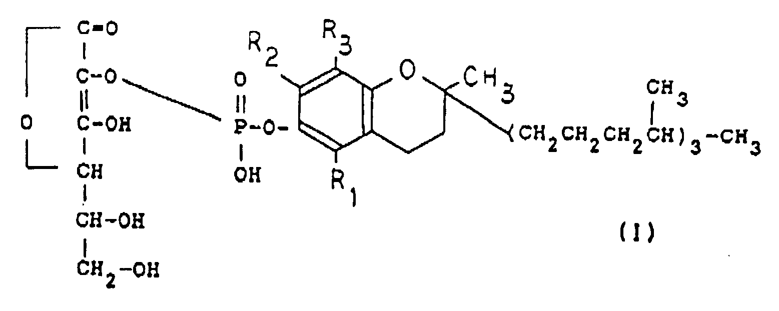 dans laquelle R₁, R₂ et R₃ désignent indépendamment l'un de l'autre un atome d'hydrogène ou un groupe méthyle, ou d'un sel correspondant pharmaceutiquement acceptable, pour la préparation d'une composition pharmaceutique de traitement et de prophylaxie du corps humain présentant des troubles de neuropathie ou d'arthrosclérose résultant de complications diabétiques lorsque ledit corps humain développe la réaction de Maillard.
